# FASCICULE DE BREVET EUROPEEN

(11) **EP 2 547 248 B1**
(45) Date de publication et mention de la délivrance du brevet: **10.05.2017**
(21) Numéro de dépôt: 11709387.2
(22) Date de dépôt: 18.03.2011
(51) Int. Cl.: A61B 3/11, A61B 3/14

(54) **PROCÉDÉ DE MESURE DE DISTANCE INTER-PUPILLAIRE**
VERFAHREN ZUR MESSUNG EINER INTERPUPILLÄREN DISTANZ
METHOD FOR MEASURING AN INTERPUPILLARY DISTANCE

(30) Priorité: 19.03.2010 FR 1052001
(43) Date de publication de la demande: 23.01.2013
(73) Titulaire: Fittingbox, 31670 Labege (FR)
(72) Inventeur: CHOUKROUN, Ariel, F-31000 Toulouse (FR)
(74) Mandataire: Ipside
(86) Numéro de dépôt international: PCT/EP2011/054138
(87) Numéro de publication internationale: WO 2011/113936

(56) Documents cités:
- FR-A1- 2 620 927
- FR-A1- 2 688 679
- FR-A1- 2 914 173
- FR-A3- 2 663 528
- GB-A- 2 449 855

## Description

La présente invention relève du domaine de l'optique et plus précisément de l'ophtalmologie.

### Contexte de l'invention et problèmes posés

Lorsque une personne (nommée sujet dans la suite du texte) cherche à acquérir des lunettes, la première étape est naturellement la détermination des caractéristiques des verres nécessaires à la correction de sa vue. Cette étape est réalisée par un ophtalmologiste. L'étape suivante est le choix d'une monture qui corresponde aux désirs du sujet.

L'opticien en charge de cette personne doit enfin, en vue de la fabrication effective de la paire de lunettes équipée des verres correcteurs, prendre une série de mesures pour adapter les lunettes (verres et monture) à la morphologie précise du sujet.

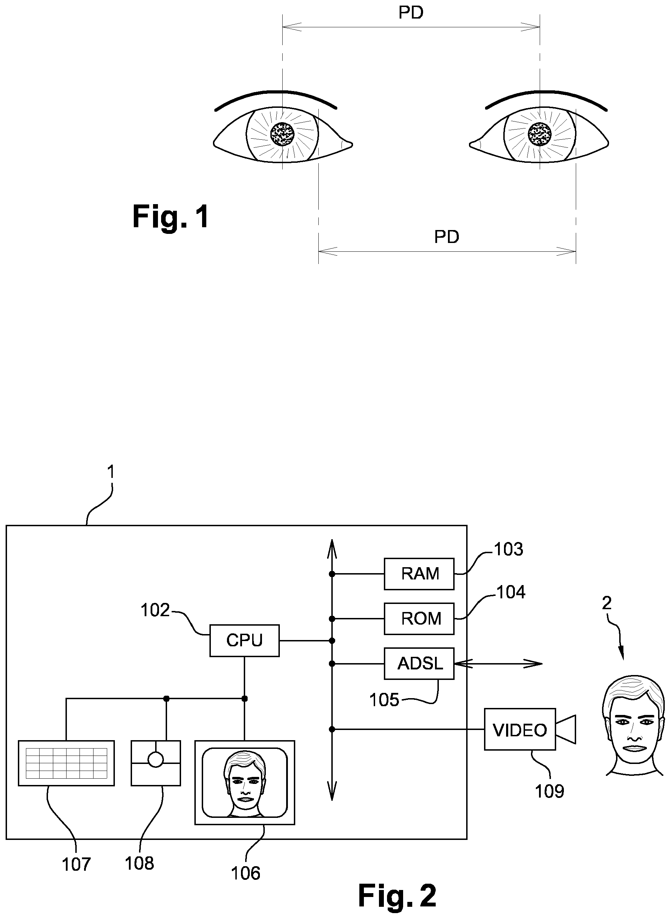

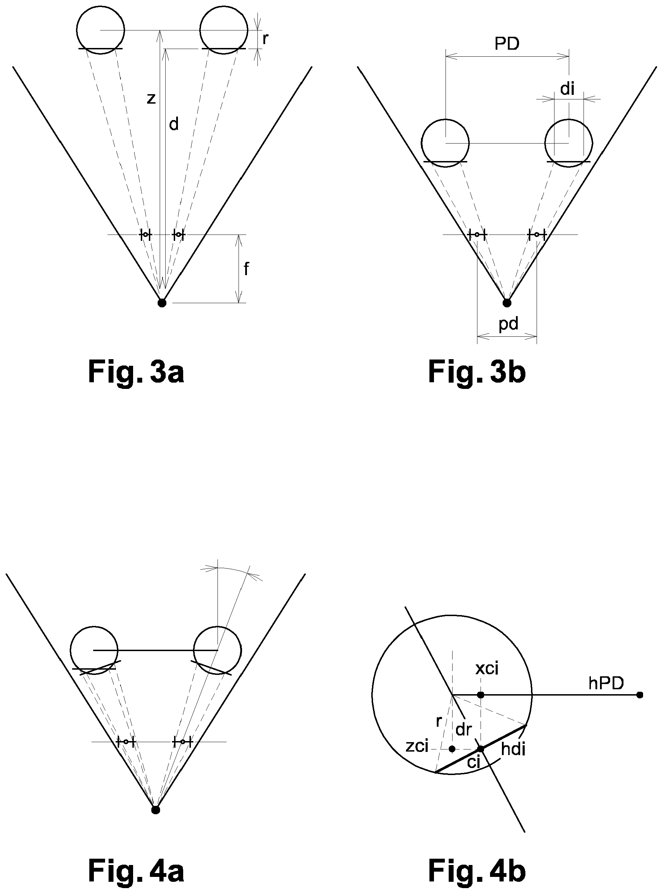

Parmi ces mesures, il est clair que la distance inter-pupillaire, notée **PD** dans la suite de la description (dite **"Pupil Distance Measurement"**, ou "P.D. measurement" en langue anglaise), définie comme la distance qui sépare les centre des pupilles des deux yeux (voir figure 1 et figure 3b) lorsque le sujet est parfaitement de face, la tête droite et regarde à l'infini, est une mesure clé. Elle est aussi appelée **"PD far"** pour caractériser le fait que le sujet regarde à l'infini. Par ailleurs, les praticiens de l'optique utilisent aussi une mesure de regard près (« **PD near** ») et une mesure intermédiaire.

Cette mesure de distance inter-pupillaire PD détermine en effet la distance relative des axes optiques des verres montés sur les lunettes. On rappelle que chacun de ces verres est, de façon simplifiée, géométriquement conformé comme l'espace compris entre deux calottes sphériques de centres différents.

Une mesure correcte de la distance inter-pupillaire PD est déterminante pour une vision parfaitement corrigée. Au contraire, une mesure incorrecte provoque un désalignement d'au moins un des verres par rapport à l'axe optique de l'oeil situé face au verre, avec de façon résultante une vision imparfaite et une fatigue oculaire.

La distance moyenne entre les centres des pupilles est de 64 millimètres pour les adultes, avec 80% des valeurs comprises entre 62 et 68 mm. La précision nécessaire est de l'ordre de 0,5 mm pour une vision correcte, particulièrement pour ce qui est des verres progressifs, qui doivent être placés le plus parfaitement possible vis à vis des yeux du sujet. On note qu'avec le vieillissement progressif de la population, la part de verres progressifs augmente dans le nombre total de verres correcteurs prescrits, et dépasse à ce jour 20% du total.

En général la mesure de distance inter-pupillaire PD effectuée est le "PD far" réalisée avec un pupillomètre ou par un autre procédé. La mesure supposée est donc la distance entre les droites passant par les centres optiques et les centres des pupilles et supposées parallèles. Cela repose sur quatre hypothèses : que le sujet regarde bien à l'infini, que l'instrument de mesure prenne en compte la projection orthographique et soit suffisamment précis et qu'il n'y ait pas de problème de vergence du sujet.

Dans la pratique, l'expression de la distance PD tient compte de la **vergence** des sujets : par exemple, regarder à l'infini requiert de manière générale d'avoir les droites précédemment définies divergentes (non parallèles) à cause du système physique de l'oeil (cornée) et de perception (intérieur de l'oeil, cerveau).

De façon corrélative, deux mesures supplémentaires sont également souhaitables, pour déterminer la façon de porter les lunettes du sujet, ainsi que la morphologie de son visage.

La première mesure est connue sous le nom de "**mono-PD**", et représente la distance du centre de la pupille au centre de la monture (qui matérialise le point d'appui des lunettes sur le nez) lorsque le sujet regarde à l'infini.

La deuxième mesure est connue sous le nom de « **Fitting Cross Heights** » (en langue anglaise), utilisée dans le cadre de verres progressifs ("varifocals"). Cette mesure est celle de la hauteur de la ligne reliant les deux centres des pupilles par rapport à un niveau de référence des lunettes (par exemple le bas des lunettes). Comme on le comprend également, une mesure précise de cette hauteur est préférable pour que les lunettes soient adaptées à une vue à l'infini correcte.

Il existe plusieurs méthodes classiques pour réaliser la mesure de distance inter-pupillaire PD. La mesure de distance inter-pupillaire peut être réalisée entre les deux yeux (mesure binoculaire), ou par un calcul de distance entre le centre d'une pupille et le centre des lunettes (mesure monoculaire).

L'une des méthodes de l'art antérieur est la simple mesure avec un double décimètre de l'espace entre les bords des iris des deux yeux, en se regardant dans un miroir. Cette mesure, approximative, donne une précision de l'ordre de 3 millimètres, suffisante pour les verres correcteurs classiques, mais inadaptée aux verres progressifs.

Une autre solution, utilisée en général par les opticiens, consiste à positionner le visage du sujet sur un repère mécanique fixe, comportant schématiquement des oculaires et une molette, et à effectuer une mesure optique. Ce procédé, plus précis que le précédent, souffre cependant d'une imprécision qui fait qu'un même sujet peut se voir trouver des distances inter pupillaires PD variant de quelques millimètres selon les opticiens effectuant la mesure.

Encore une autre méthode consiste à prendre au moins une image du sujet et d'un objet de calibrage, et à déduire la distance inter-pupillaire PD de la valeur mesurée sur l'image et de coefficients correctifs déterminés d'après l'image de l'objet de calibrage. Ce procédé souffre également de demander une position prédéterminée du sujet, difficile à obtenir en pratique.

Les documents FR 2 914 173, FR 2 688 679 et FR 2 663 528 décrivent des appareils et des méthodes de mesure de la distance inter-pupilllaire.

La mesure de "Fitting Cross Height" est, quant-à-elle, réalisée de manière plus ou moins empirique par les opticiens en observant le port des lunettes du sujet.

Par ailleurs, ces mesures supposent que le sujet regarde effectivement à l'infini, ce qui est rarement le cas, et peut perturber la mesure de un à deux millimètres également.

Enfin, il est connu que l'oeil humain n'est pas une sphère parfaite, et que son mouvement n'est pas modélisable par une simple rotation, mais par une combinaison de rotation et de translation, ce qui fait que la mesure doit tenir compte de l'orientation du regard au moment de ladite mesure, sous peine de ne pouvoir être extrapolée à la position normale de regard à l'infini.

On comprend donc que la mesure est rendue difficile, non seulement par les équipements et procédés existants, mais par la nature même du corps humain et les mouvements inévitables ou erreurs de positionnement du sujet

Pour les systèmes optiques actuels, on utilise en général un simple report de distance. De même, lors de l'utilisation d'un système optique photo: on observe un décentrage mesuré du à la perspective de la caméra et non à la morphologie. Pour une mesure plus juste, il faut donc connaitre la position tridimensionnelle et la morphologie du système visuel du sujet. Pour la mesure, il est admis par l'homme du métier que le centre de rotation de l'oeil est le point focal de formation de l'image.

Il existe des systèmes de mesure en trois dimensions, mais ceux-ci sont très invasifs et nécessitent d'être mis en oeuvre dans un magasin doté de matériel sophistiqué et onéreux.

**Objectifs de l'invention**L'objectif de cette invention est alors de proposer un procédé de mesure de distance inter-pupillaire qui remédie aux problèmes d'imprécision et de difficulté de mesure précédemment cité.

Selon un second but de l'invention, le procédé peut être mis en oeuvre à coût réduit, ce qui permet d'envisager une large utilisation.

Un troisième but de l'invention est de fournir une mesure de distance inter-pupillaire en un temps très court.

Un autre but de l'invention est de pouvoir également être mise en oeuvre sans nécessiter d'objets de calibrage.

Le procédé de mesure peut être mis en oeuvre avec un matériel généralement disponible dans le grand public, et utilise un protocole à la portée de tous : ce qui évite à un utilisateur de devoir se rendre en magasin.

### Exposé de l'invention

A cet effet, l'invention concerne un procédé d'estimation de distance inter-pupillaire (PD) tel que défini dans la revendication 1.

Selon divers modes de mise en oeuvre, éventuellement utilisés en conjonction :
- la phase 100 comprend des étapes suivantes :
   étape101 : placement de la personne face à la caméra, à une distance préalablement choisie de la caméra,
   étape 102 : mouvement du visage de la personne vers la caméra en la fixant,
   étape 103 : arrêt quand la personne ne peut plus fixer l'objectif ou quand elle reçoit un signal d'arrêt.
- la distance préalablement choisie étant sensiblement la longueur d'un bras.

L'étape 102 peut être remplacée par d'autres mouvements qui permettent de distinguer le mouvement des yeux de celui du visage :
La personne fixe la caméra et tourne la tête de gauche à droite.
La personne garde la tête fixe et regarde à différents endroits.

La personne regarde la caméra, puis à l'infini

La personne regarde un ensemble de points prédéfinis sur l'écran.
- la phase 200 comprend des étapes suivantes :
   étape 201 : apprentissage de mesures statistiques,
   étape 202. : caractérisation des mesures images,
   étape 203 : détermination statistique des données 3D, et des paramètres recherchés,
   étape 204 : optimisation non-linéaire des paramètres.
- les paramètres recherchés comprennent la distance inter-pupillaire *PD,* le diamètre de l'iris *di,* la focale de la caméra *f*, les distances *zi* (i=1 ...n) de la caméra à la personne pour les images successives acquises lors du mouvement.
- l'étape 201 comprend des sous-étapes suivantes :
   201a : mesure d'un ensemble de données morphologiques et mécaniques,
   201b : construction d'une scène de projection du système PD-iris présenté, et fabrication d'une table de correspondance qui échantillonne la réalité continue des paramètres selon les entrées suivantes, et pour des tailles d'images considérées : *PDpx, dipx, PD, di, f, z* avec *PDpx* et *dipx* les mesures image des données *PD* et *di,*
   201c : établissement de plusieurs densités de probabilités discrètes lissées par une méthode de noyau et réparties dans les tables exprimant entre autres les fonctions suivantes :
      i. *PD* = *f (PDpx, dipx, f, zi)*
      ii. *di* = *f (PDpx, dipx, f, zi)*
- l'étape 202 comprend des sous-étapes suivantes :
   202a : détection de visage,
   202b : détection et reconnaissance des yeux,
   202c : description fine des iris par estimation des paramètres du cercle ou de l'ellipse représenté par la projection de l'iris sur l'image,
   202d : déduction des valeurs *PDpx,* distance entre les deux centres des images des iris, et *dipx,* diamètres des images des iris.
- l'étape 203 comprend des sous-étapes suivantes :
   203a : sous l'hypothèse du mouvement à vitesse constante, expression de l'ensemble des paramètres *zi* avec deux paramètres : *z0,* la distance de départ (la plus loin) et *dz,* la distance entre deux prises de vue,
   203b : simulation d'un ensemble de réalisations d'échantillonnage du mouvement afin d'expliquer au mieux et de manière probabiliste les paramètres *f*, *PD* et *di* par rapport aux mesures observées dans les images *PDpx* et *dipx.*
- la sous-étape 203b comporte des points suivants :
   i. pour un ensemble de triplets de réalisation (*z0*, *dz*, *f)* tels que les *zi* (i=1...n) et *f* soient compris dans une plage de valeurs acceptables pour le protocole, on établit un histogramme des triplets pour lesquels on a le plus de réponses de couples (*PD*, *di*) grâce aux fonctions de correspondance, et on fait varier les réalisations de (*zi, PDpx, dipx*) autour des valeurs considérées afin de simuler les lois de réalisation des mesures,
   ii. on retrouve la focale *f* pour le pic maximum des probabilités construites,
   iii. de *f*, on déduit *PD* et di grâce aux mêmes tables, comme la moyenne des propriétés de convergence normale asymptotique des expériences menées de l'ensemble des *PD* et di retrouvés,
   iv. on retrouve le couple (*z0, dz)* pour lequel les paramètres ci-dessus sont les plus probables.
- l'étape 204 comprend des sous-étapes suivantes :
   204a : relâchement des contraintes de distance constante entre les *zi* et initialisation avec la solution précédente,
   204b : définition des confiances sur les paramètres 3D par la recherche de l'ensemble des valeurs *zi, PD, di, f* qui permettent de minimiser l'erreur de re-projection des iris sur les images acquises,
   204c : utilisation d'un algorithme du simplexe de type Nelder-Mead / ou un algorithme de recalage de texture additif.

Selon une mise en oeuvre avantageuse, le procédé comporte une phase 300 de calibrage de la caméra (l'estimation sa distance focale *f*) utilisée pour les acquisitions de données,
ledit calibrage comportant l'utilisation d'un objet prédéterminé et en le présentant devant la caméra, sous diverses distances et orientations angulaires, ledit objet comportant plusieurs cercles concentriques sur un même plan permet de calibrer une caméra ou deux cercles coplanaires.

Selon un mode de mise en oeuvre préféré, l'objet est un disque compact (CD).

Avantageusement, la phase 300 comporte des étapes suivantes :
étape 301 : présentation et déplacement de l'objet devant la caméra,
étape 302 : estimation du centre de l'objet,
étape 303 : détection de contour radial et obtention d'une image de contours avec les contours chainés principaux de l'image,
étape 304 : obtention d'une texture contour/carte de distances au contour de l'objet de face,
étape 305 : utilisation d'un algorithme d'alignement de texture sur l'image des contours pour déterminer les paramètres de calibrage recherchés.

Selon une autre mise en oeuvre avantageuse, la personne se déplace en plaquant sur sa joue un objet de taille connue, par exemple une pièce de monnaie.

Cet objet permet de fixer les valeurs *z0*, *zi* et *dz* recherchées.

Lorsqu'elle est couplée à la première solution avantageuse avec calibrage de caméra, cette solution réduit l'incertitude sur la précision de la mesure.

L'invention vise également un produit programme d'ordinateur, comprenant des instructions de code de programme pour l'exécution d'un procédé tel qu'exposé, lorsque ledit programme est exécuté sur un ordinateur.

L'invention vise sous un autre aspect un dispositif de mesure d'estimation de distance inter-pupillaire (PD) d'une personne comportant :
- des moyens d'acquisition de données, lors d'un mouvement prédéterminé de la personne face à une caméra,
- des moyens de calcul de paramètres morphologiques et dimensionnels sur la base des données acquises.

### Brève description des figures

La description qui va suivre, donnée uniquement à titre d'exemple d'un mode de réalisation de l'invention, est faite en se référant aux figures annexées dans lesquelles :
- la figure 1 illustre la distance inter-pupillaire que l'on cherche à mesurer,
- la figure 2 illustre schématiquement les éléments mis en oeuvre dans le procédé selon l'invention,
- les figures 3a et 3b illustrent les paramètres, dont la distance inter-pupillaire, que l'on cherche à mesurer, dans le cas d'un mouvement de l'utilisateur vers la caméra en regardant à l'infini, la figure 1a avant le mouvement, et la figure 1b après ce mouvement,
- la figure 4a illustre de manière analogue la situation des yeux, à la fin d'un mouvement de l'utilisateur vers la caméra en fixant celle-ci, et la figure 4b est un détail d'un oeil et des paramètres de mesure associés,
- la figure 5 illustre les étapes de la phase d'acquisition de données du procédé de mesure conforme à un mode de mise en oeuvre de l'invention,
- la figure 6 illustre les étapes de la phase de calcul du procédé de mesure conforme à un mode de mise en oeuvre de l'invention,
- la figure 7 illustre les étapes d'une phase de calibrage de caméra utilisée dans une variante du procédé.

### Description détaillée d'un mode de réalisation de l'invention

Le procédé selon l'invention est destiné à être mise en oeuvre sous forme de programme d'ordinateur comprenant une série de lignes d'instructions de codes de programme pour l'exécution des étapes du procédé lorsque ledit programme est exécuté sur un ordinateur.

L'ordinateur dont il est question est par exemple un micro-ordinateur de type PC.

Comme on le voit sur la figure 2, le dispositif de mesure mettant en oeuvre le procédé selon l'invention comporte donc un micro-ordinateur 1, utilisé par un opérateur 2, confondu dans le présent exemple non limitatif avec le sujet de la mesure de distance inter-pupillaire, ledit micro-ordinateur 1 comportant par exemple, reliés entre eux par un bus d'adresses et de données 101 :
- une unité centrale 102 ;
- une mémoire vive RAM 103 ;
- une mémoire morte 104;
- une interface réseau 105, par exemple un modem ADSL classique ;
et, indépendamment du bus 101 :
- un écran de visualisation 106 ;
- un clavier 107 ;
- une souris 108.

La mémoire vive 103 constitue un moyen de stockage d'information lisible par un ordinateur ou un microprocesseur. Elle conserve des données, des variables et des résultats intermédiaires de traitement.

La mémoire morte 104 constitue un moyen de stockage d'information lisible par un ordinateur ou un microprocesseur. Elle conserve des instructions d'un programme informatique qui permet la mise en oeuvre du procédé objet de l'invention.

Selon une variante, la mémoire morte 104 est amovible, partiellement ou totalement, et comporte, par exemple un compact disque à mémoire figée ("CD-ROM" en anglais).

Le dispositif comporte également un dispositif d'acquisition d'images fixes, par exemple caméra vidéo 109, de type classique, reliée à l'ordinateur 1 par le bus de données 101.

Chacun des éléments illustrés en figure 2 est bien connu de l'homme du métier. Ces éléments ne sont donc pas décrits ici.

L'unité centrale 102 chez l'opérateur est adaptée à mettre en oeuvre les organigrammes décrits plus bas.

### Modèle de calcul et dispositif

Tel que décrit ici dans un exemple de mise en oeuvre non limitatif, l'invention comprend un système de prise de vue et un protocole associé. Ceux-ci permettent d'estimer avec une précision contrôlée et de qualité égale aux instruments de mesure classique (typiquement erreur < 1 mm) la distance inter-pupillaire d'une personne. Ils permettent par ailleurs de déterminer les paramètres de la caméra et les distances auxquelles se trouve la personne quand les images sont acquises par la caméra.

Pour cela, on considère le système suivant yeux-iris, illustré en figures 3a et 3b et paramétré par les variables suivantes :
- *r* : rayon du globe oculaire
- *di* : diamètre de l'iris, *hdi* : rayon de l'iris
- *PD* : distance inter-pupillaire, *hPD* : demi-distance entre les yeux
- *z* : distance du centre des yeux à la caméra
- *d* : distance du centre des iris à la caméra
- *f* : distance focale de la caméra

Le système global de prise de vue comprend :
- un ordinateur
- un écran
- au moins une caméra (par exemple de type "webcam") ou un appareil photo située éventuellement au dessus de l'écran.

L'utilisateur se place centré, de telle sorte à être filmé par la caméra et se voir de face sur l'image enregistrée par la caméra et rediffusée sur son écran.

Pour ce système, on peut mesurer dans le plan image la position des iris projetés ainsi que leur forme et leur taille.

Afin de retrouver ces paramètres, on peut favorablement utiliser un ensemble d'informations dites "connaissances a priori". Parmi celles-ci, on peut citer par exemple :
1. incertitude limitée sur les paramètres morphologiques :
   ∘ *PD* : *sigma_PD*
   ∘ *di* : *sigma_di*
   ∘ *r* : *sigma_r*
2. mouvement contrôlé
3. focale déterminée
   ∘ par une méthode de calibrage décrite ci-après
   ∘ par les connaissances techniques de fabrication

On propose des solutions, à chaque ajout de connaissances a priori de type 1 2 ou 3, avec une précision accrue.

L'utilisation d'un mouvement prédéterminé de l'utilisateur permet d'acquérir plusieurs images afin de déterminer les paramètres invariants entre les prises de vue. A chaque prise de vue, une nouvelle distance *zi* est à estimer.

On propose à l'utilisateur situé de se déplacer selon le mouvement suivant :
1. s'avancer vers (ou s'éloigner de) la caméra en regardant à l'infini :
   a. les iris peuvent être considérés sur un même plan
   b. Il est possible de les repérer précisément selon une méthode par exemple de type AAM [Cootes98].
   c. Le calibrage de la caméra se fait grâce à une méthode, décrite plus loin, qui permet de considérer deux cercles sur un même plan, les iris, et de mesurer la déformation de leur projection en ellipses en fonction de leur position et de la distance focale.
   d. La focale f est alors estimée par l'apparence des iris.

   Dans une variante de mise en oeuvre, éventuellement utilisée conjointement à la précédente, le procédé utilise le protocole de mouvement de l'utilisateur suivant :
2. s'avancer vers (ou s'éloigner de) la caméra en fixant l'objectif de ladite caméra :
   a. les yeux tournent d'un angle dépendant de la distance à la caméra. La projection des iris est en pratique presque toujours assimilable à un cercle.
   b. Cette invariance projective du mouvement dans l'image permet que l'apparence du modèle est essentiellement dépendante du couple (*PD*/*di*) quelle que soit la compensation des valeurs *f* et *z*.

Les figures 4a et 4b montrent les nouveaux paramètres utilisés dans ce cas.

Dans les deux cas de mouvement, celui-ci permet de ne pas avoir besoin d'estimer les paramètres de positionnement 3D *tx ,ty ,rx ,ry* du système dans un premier temps.

### Procédé de calcul

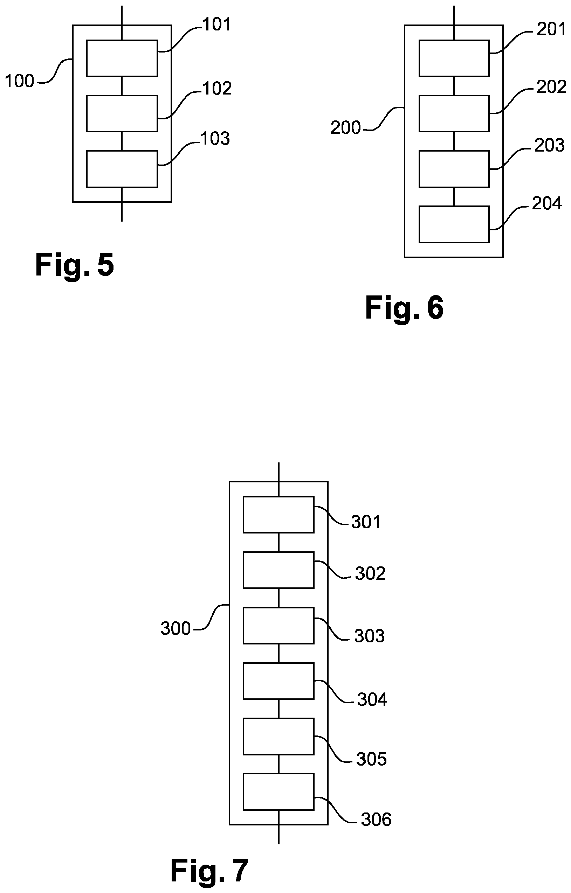

Le procédé comporte tout d'abord une phase 100 d'acquisition de données (voir figure 5) utilisant un mouvement prédéterminé de l'utilisateur (s'avancer vers ou s'éloigner de la caméra):
étape 101. L'utilisateur se place à une distance d'un bras de la caméra,
étape 102. L'utilisateur avance à vitesse constante son visage vers la caméra en la fixant,
étape 103. L'utilisateur s'arrête quand il ne peut plus fixer l'objectif ou quand le système lui fait signe de s'arrêter.

Les paramètres recherchés sont estimés dans une phase 200 de calcul (voir figure 6), selon le schéma suivant :
étape 201. Apprentissage de mesures statistiques
   201 a. Mesure d'un ensemble de données morphologiques *PD, di, r* et mécaniques : *f*
   201 b. Construction d'une scène de projection du système PD-iris présenté et fabrication d'une table de correspondance qui échantillonne la réalité continue des paramètres selon les entrées suivantes, et pour des tailles d'images considérées : *PDpx, dipx, PD, di, f, z*, avec *PDpx* et *dipx* les mesures image des données *PD* et *di.*
   201c. Etablissement de plusieurs densités de probabilités discrètes lissées par une méthode de noyau et réparties dans les tables exprimant entre autres les fonctions suivantes
      i. *PD* = *f (PDpx, dipx, f, zi)*
      ii. *di* = *f (PDpx, dipx, f, zi)*
étape 202. Caractérisation des mesures images
   202a. Détection de visage, par exemple suivant l'algorithme de Viola et Jones, connu en soi et non détaillé ici.
   202b. Détection et reconnaissance des yeux, par exemple grâce à un modèle de type AAM [Cootes98].
   202c. Description fine des iris par estimation des paramètres du cercle ou de l'ellipse représenté par la projection de l'iris sur l'image.
   202d. Déduction (sous-pixellique, c'est-à-dire meilleure que la valeur d'un pixel, grâce à l'estimation paramétrique des images iris) des valeurs *PDpx,* distance entre les deux centres des images des iris, et *dipx,* diamètres des images des iris.
étape 203. Détermination statistique des données 3D, paramètres recherchés : *PD, di, f, zi* (i=1 ...n).
   203a. Sous l'hypothèse du mouvement à vitesse constante, l'ensemble des paramètres *zi* va être exprimé avec deux paramètres : *z0,* la distance de départ (la plus loin) et *dz,* la distance entre deux prises de vue. Ces deux paramètres définissent complètement l'ensemble des *zi* pour les prises de vue. Les *zi* représentent un échantillonnage du mouvement. Un des objets de l'algorithme est de les déterminer.
   203b. On simule un ensemble de réalisations d'échantillonnage du mouvement afin d'expliquer au mieux et de manière probabiliste les paramètres *f*, *PD* et *di* par rapport aux mesures observées dans les images *PDpx* et *dipx.*
      i. Pour un ensemble de triplets de réalisation (*z0*, *dz*, *f*) tels que les *zi* (i=1...n) et *f* soient compris dans une plage de valeurs acceptables pour le protocole, on établit un histogramme des triplets pour lesquels on a le plus de réponses de couples *(PD, di)* grâce aux fonctions de correspondance. On fait varier aussi les réalisations de (*zi, PDpx, dipx)* autour des valeurs considérées afin de simuler les lois de réalisation des mesures.
      ii. On retrouve la focale *f* pour le pic maximum des probabilités construites.
      iii. De *f*, on déduit *PD* et *di* grâce aux mêmes tables, comme la moyenne (propriétés de convergence normale asymptotique des expériences menées) de l'ensemble des *PD* et *di* retrouvés.
      iv. On retrouve le couple (*z0, dz*) pour lequel les paramètres ci-dessus sont les plus probables.
étape 204. Optimisation non-linéaire des paramètres
   204a. On relâche les contraintes de distance constante entre les *zi.* On initialise avec la solution précédente.
   204b. On définit des confiances sur les paramètres 3D. On cherche l'ensemble des *zi, PD, di, f* qui permettent de minimiser l'erreur de re-projection des iris sur les images acquises.
   204c. On utilise un algorithme du simplexe, par exemple de type Nelder-Mead ou un algorithme de recalage de texture additif.

On obtient finalement les valeurs de paramètres morphologiques et dimensionnels définissant la distance inter-pupillaire *PD,* la focale *f* de la caméra, la distance de l'utilisateur à la caméra et la position relative de ses yeux par rapport à cette caméra. En particulier, le procédé permet de déterminer la distance inter-pupillaire de l'utilisateur avec une précision contrôlée et de qualité égale aux instruments de mesure classique (typiquement une erreur inférieure à un millimètre).

Ceux-ci permettent notamment de positionner de façon correcte une image d'un modèle de paire de lunette virtuelle sur l'image du visage de l'utilisateur, acquise par la caméra. Le procédé garantit l'apparence des rapports entre les tailles du visage utilisateur et des lunettes dans l'image. Le procédé permet d'immerger des modèles numériques 3D de lunettes issus d'objets réels dans la scène, à la distance exacte nécessaire pour permettre un essayage métriquement correct pour n'importe quelle photo, quelle que soit alors la position du visage face à la caméra.

De même, la mesure exacte de la distance inter-pupillaire permet la fabrication de lunettes adaptées à être portées par l'utilisateur, et dont les verres sont précisément positionnés optiquement face aux yeux de l'utilisateur.

En résumé, le procédé objet de la présente demande mesure la distance inter-pupillaire en trois dimensions PD 3D et la distance mono PD 3D de façon indépendante du système de prise de vue.

En effet, dans ce procédé, on mesure la distance métrique entre les positions 3D des points focaux et non des projections arbitraires utilisées dans les systèmes courants.

Grâce à ce procédé il est possible de retrouver l'ensemble des mesures classiques de PD (à savoir PD « near » et PD « far ») ainsi que le modèle de vergence spécifique à chaque sujet. Cela a un intérêt pour la construction et le montage des verres adaptés au parcours de l'oeil et non pas à une seule ou un nombre fini de directions du regard. Cette mesure de PD 3D est donc plus pertinente pour la monture des verres et pour la précision de découpe des verres que les mesures classiques de PD.

### Variantes de l'invention

La portée de la présente invention ne se limite pas aux détails des formes de réalisation ci-dessus considérées à titre d'exemple, mais s'étend au contraire aux modifications à la portée de l'homme de l'art.

Dans une variante de mise en oeuvre, le calibrage de la caméra est effectué en utilisant un objet de type disque compact (CD) et en le présentant devant la caméra, éventuellement sous diverses distances et orientations angulaires.

Dans la suite, par calibrage de la caméra, on entend l'estimation sa distance focale f. Les autres paramètres du modèle sténopé sont supposés connus de manière satisfaisante pour l'application. Par exemple, le capteur de la plupart des caméras numériques modernes est constitué de pixel carrés, et le centre optique peut être raisonnablement fixé au centre du capteur (ie de l'image).

Ce calibrage se base ici sur des résultats connus de l'homme du métier dont notamment :
- Un objet comportant plusieurs cercles concentriques sur un même plan permet de calibrer une caméra
- Deux cercles coplanaires permettent de calibrer une caméra

On applique alors ces principes et on choisit d'utiliser un objet que tout le monde possède, tel que par exemple un CD ou une pièce de monnaie qui comporte des cercles concentriques.

On propose deux approches :
- Solution algébrique : elle utilise des propriétés géométriques pour obtenir un résultat algébrique. Elle valide les aspects théoriques.
- Solution par alignement 3D : elle utilise une modélisation 3D du système. On utilise un processus d'alignement 3D qui permet de calibrer la caméra quelque soit l'objet 3D connu, pourvu qu'il possède un plan sur lequel certaines dimensions sont connues.

Cette dernière approche est très robuste : elle permet de fonctionner dans beaucoup de conditions d'illuminations et est peu sensible aux occultations. Elle permet de retrouver l'ensemble des paramètres 3D de la scène : position et orientation de l'objet, paramètres de la caméra (distance focale en particulier).

La description qui suit concerne une phase 300 de calibrage de la caméra mettant en oeuvre une approche avec un CD. Elle comporte des étapes suivantes (voir figure 7):
étape 301 : présentation et déplacement du CD devant la caméra
étape 302 : estimation du centre du CD : clic utilisateur ou détection de la main,
étape 303 : détection de contour radial par exemple par une méthode utilisant un filtre Canny-Deriche. On obtient une image de contours avec les contours chainés principaux de l'image,
étape 304 : on dispose d'une texture contour/carte de distances au contour du CD de face.

On peut exprimer l'homographie qui relie la texture et le CD orienté et positionné dans la scène 3D.

étape 305 : utilisation d'un algorithme d'alignement de texture sur l'image des contours pour retrouver précisément les paramètres recherchés

Pour obtenir la solution initiale de cet algorithme, on peut dans une étape 306 utiliser la méthode algébrique, ou un ensemble d'estimations linaires en fixant certains paramètres.

Un tel algorithme de recalage de texture additif peut être utilisé dans le cadre de l'étape 204c décrite plus haut.

Dans des variantes de mise en oeuvre, éventuellement utilisées en conjonction avec la méthode décrite plus haut, on utilise d'autres connaissances a priori :
- Focale *f* : on peut obtenir des "connaissances a priori" des paramètres de caméra (focale *f*) en repérant le modèle de caméra. Pour chaque caméra connue, un calibrage a été effectué préalablement, par exemple en utilisant la méthode décrite ci-dessus et mémorisé. Si la caméra est inconnue, on effectue le calibrage comme décrit ci-dessus.
- Distances *zi* : l'utilisateur avance vers la caméra avec une pièce de monnaie disposée contre son visage au niveau de son oeil, sensiblement dans le plan de ses yeux.
- Longueur du bras. On peut demander à l'utilisateur de se positionner à une longueur de bras de la camera. Grâce aux données statistiques morphologiques, en connaissant le genre et la taille de la personne, on peut en déduire la longueur moyenne de son bras. Cela permet de donner la valeur de première distance *zi*.

D'autres protocoles sont également envisageables en variante.

La détermination des paramètres *f*, *PD* et *z* des yeux peut être réalisée avec une seule photographie de face d'une personne tenant un CD dont une extrémité est collée contre le bord de son oeil.

Ce protocole ne constitue pas un simple report de mesures images du CD dont certaines distances sont connues, mais comporte les opérations suivantes :
- Estimation de *f* grâce à la détection des différentes ellipses qui sont les projections des bords du CD.
- Estimation du plan de *f* et de la profondeur *z* du point de contact *f* avec le visage.

Dans encore une autre variante, deux caméras vidéo sont utilisées, par exemple de type webcam, et les images obtenues par les deux sources sont utilisées par le procédé, autrement inchangé. Cette variante permet un gain de temps d'acquisition des paramètres du visage. Elle permet de calculer le mouvement réellement parcouru *dz* entre les vues, et offre donc plus de précision dans l'estimation.

Il est clair que le procédé décrit pourrait être appliqué au placement d'une image d'un autre objet de dimensions connues sur le visage de l'utilisateur, dès lors que l'on connait sa distance à la caméra et des dimensions principales de son visage, telles que déterminées par un procédé tel que décrit ci-dessus.

Il est à noter que l'invention proposée ne se limite pas, contrairement aux autres méthodes optiques, à un report de mesure d'un objet connu sur des mesures images à effectuer. Il s'agit bien d'une estimation 3D métrique des paramètres recherchés dans l'espace réel du sujet.

Connaissant la mesure PD 3D, il est possible de retrouver les mesures PD "classiques" par un protocole additionnel. En effet, en utilisant une seule photo supplémentaire du sujet regardant à l'infini, dans laquelle les yeux sont visibles, un recalage 3D du système visage/yeux sur l'image permet de retrouver la vergence à l'infini propre au sujet et d'en déduire la valeur de PD « far ».

De même si le sujet regarde à une distance définie on peut calculer la valeur de PD pour cette distance (le PD « near » peut donc être déduit). Le corollaire de ce résultat est l'expression d'un modèle de vergence de chaque oeil du sujet dont les échantillons sont le nombre d'acquisitions à des distances différentes.

Dans la méthode précédemment détaillée, les mesures d'apprentissage sont générées grâce à des données 3D. Ce sont donc des données 3D métriques qui sont retrouvées. Une autre façon de procéder, décrite ci-après, reconstruit le système 3D complet : globes oculaires-visage-iris depuis les mesures image effectuées.

### Système de reconstruction 3D et mesure de distance inter pupillaire PD.

Dans ce protocole, on retrouve les centres de rotation des globes oculaires. Ces centres représentent les centres de vision autour desquels les yeux tournent et qui sont le centre optique du système oeil. On définit la distance inter pupillaire PD 3D comme la mesure métrique 3D de la distance entre les deux centres de rotation. Conjointement, l'ensemble des paramètres 3D de la scène sont retrouvés. Ce protocole suppose que l'on travaille en connaissant uniquement la valeur de la focale de la caméra et la profondeur z du visage ou des dimensions principales du visage. Ces valeurs peuvent être obtenues par calibrage ou supposées.

Le principe est le suivant :
A partir des n images acquises, on récupère par détection ou suivi 2D un ensemble d'indices images suivants :
   1. Points d'intérêt du visage permettant d'obtenir le mouvement rigide du visage.
   2. Contours des iris permettant d'obtenir le mouvement global subi par les globes oculaires (mouvement rigide du visage + rotations des globes oculaires).
   3. Points ou contours décrivant la frontière globles oculaires/visage (comme par exemple les commissures des yeux ou encore les contours des paupières sur les globes oculaires). Ces points ou contours subissant uniquement le mouvement rigide du visage et reliant le visage aux globes oculaires permettent de dé-corréler le mouvement rigide de la rotation des globes oculaires et permettent donc de reconstruire dans l'espace réel les courbes 3D du mouvement de rotation des globes (leur trajet).

Les indices images sont mis en correspondances et représentent un ensemble de points 3D ou courbes 3D recherchés.

On cherche à retrouver les coordonnées 3D des centres de rotation de chaque oeil *Og* et *Od*.

### Soit n le nombre d'images acquises et i le numéro d'image courante.

Soit m le nombre de points d'intérêt du visage et j l'indice du point courant.

Soit *Pv_j* le point d'intérêt 3D recherché et *pv_ij* son indice image associé (projection).

Soit p le nombre de points d'intérêts décrivant la frontière globes/visage et k l'indice du point courant.

Soit Pe_k le point d'intérêt 3D décrivant la frontière globes/visage et pe_ik son indice image associé.

Soit q le nombre de points contours de l'iris et I l'indice du point courant.

Soit Pg_l le point contour de l'iris et pg_il son indice image associé.

On cherche :
- Le mouvement rigide *(Rx,Ry,Rz, Tx,Ty,Tz) subi par les points Pv et Pe.*
- Le mouvement combiné (mouvement rigide du visage + rotation des globes) subi par les globes oculaires et les points Pg.
- les positions des centres de rotation *Og(x,y,z)* et *Od(x,y,z)* tels que la projection du point *Cig* (resp. *Cid*) (centre 3D de l'iris à distance *dr* de *Og* resp. *Od*) qui suit la rotation de l'oeil *Re(Rex,Rey,Rez)* soit la plus proche des indices images correspondants.

La résolution de ce système se fait grâce à une minimisation aux moindres carrés entre la projection des points 3D / courbes recherchés (Pv_j, Pe_k et Pg_l) et les indices images. Cette résolution aux moindres carrés est une méthode connue de l'homme du métier, et communément appelée « bundle adjustment ». La solution initiale peut être définie grâce aux connaissances a priori de la scène comme les données statistiques sur l'oeil et le visage (tailles des globes oculaires, iris, etc.).

## Revendications

1. Procédé d'estimation de distance inter-pupillaire (PD) d'une personne, comportant :
- une phase 100 d'acquisition de données, utilisant un mouvement prédéterminé de la personne face à une caméra, cette phase 100 d'acquisition de données comportant l'acquisition d'un ensemble d'images successives représentatives des yeux de la personne, lorsque la position de cette personne évolue, entre une première configuration du visage et une seconde configuration du visage,
**caractérisé en ce qu'**il comporte en outre :
- une phase 200 de calcul de paramètres morphologiques et dimensionnels sur la base des données acquises, ladite phase 200 comprenant des étapes suivantes :
étape 201 : apprentissage de mesures statistiques, ladite étape 201 comprenant des sous-étapes suivantes :
201a : mesure d'un ensemble de données morphologiques PD, di, r et mécaniques f, où di représente le diamètre de l'iris, r le rayon du globe oculaire et f la distance focale de la caméra
201 b : construction d'une scène de projection du système PD-iris de ladite personne et fabrication d'une table de correspondance qui échantillonne la réalité continue des paramètres selon les entrées suivantes : *PDpx, dipx, PD, di, f, z* avec *z* la distance du centre des yeux à la caméra, PDpx la distance entre les deux centres des images des iris et dipx le diamètres des images des iris, *PDpx* et *dipx* correspondant aux mesures image des données *PD* et *di,*
201 c : établissement de plusieurs densités de probabilités discrètes lissées par une méthode de noyau et réparties dans les tables exprimant entre autres les fonctions suivantes :
i. *PD* = *f (PDpx, dipx, f, zi)*
ii. *di* = *f (PDpx, dipx, f, zi)* où zi (i=1 ...n) représentent les distances de la caméra à la personne pour les images successives acquises lors du mouvement, n représentant le nombre d'images
étape 202 : caractérisation des mesures images,
étape 203 : détermination statistique d'une estimation 3D métrique des paramètres recherchés dans l'espace réel du sujet : PD, di, f et zi (i=1 ...n),
étape 204 : optimisation non-linéaire des paramètres.

2. Procédé selon la revendication 1, **caractérisé en ce que** la phase 100 comprend des étapes suivantes :
étape101 : placement de la personne face à la caméra, à une distance préalablement choisie de la caméra,
étape 102 : mouvement du visage de la personne vers la caméra en la fixant,
étape 103 : arrêt quand la personne ne peut plus fixer l'objectif ou quand elle reçoit un signal d'arrêt.
- la distance préalablement choisie étant sensiblement la longueur d'un bras.

3. Procédé selon l'une des revendications 1 à 2, **caractérisé en ce que** les paramètres recherchés comprennent la distance inter-pupillaire *PD,* le diamètre de l'iris *di,* la focale de la caméra *f*, les distances *zi* (i=1 ...n) de la caméra à la personne pour les images successives acquises lors du mouvement.

4. Procédé selon l'une des revendications 1 à 3, **caractérisé en ce que** l'étape 202 comprend des sous-étapes suivantes :
202a : détection de visage,
202b : détection et reconnaissance des yeux,
202c : description fine des iris par estimation des paramètres du cercle ou de l'ellipse représenté par la projection de l'iris sur l'image,
202d : déduction des valeurs *PDpx,* distance entre les deux centres des images des iris, et *dipx,* diamètres des images des iris.

5. Procédé selon l'une des revendications 1 à 4, **caractérisé en ce que** l'étape 203 comprend des sous-étapes suivantes :
203a : sous l'hypothèse du mouvement à vitesse constante, expression de l'ensemble des paramètres *zi* avec deux paramètres : *z0*, la distance de départ (la plus loin) et *dz*, la distance entre deux prises de vue,
203b : simulation d'un ensemble de réalisations d'échantillonnage du mouvement afin d'expliquer au mieux et de manière probabiliste les paramètres *f*, *PD* et *di* par rapport aux mesures observées dans les images *PDpx* et *dipx.*

6. Procédé selon la revendication 5, **caractérisé en ce que** la sous-étape 203b comporte des points suivants :
i. pour un ensemble de triplets de réalisation (*z0*, *dz*, *f*) tels que les *zi* (i=1...n) et *f* soient compris dans une plage de valeurs acceptables pour le protocole, on établit un histogramme des triplets pour lesquels on a le plus de réponses de couples *(PD, di)* grâce aux fonctions de correspondance, et on fait varier les réalisations de (*zi, PDpx, dipx)* autour des valeurs considérées afin de simuler les lois de réalisation des mesures,
ii. on retrouve la focale *f* pour le pic maximum des probabilités construites,
iii. de *f*, on déduit *PD* et di grâce aux mêmes tables, comme la moyenne des propriétés de convergence normale asymptotique des expériences menées de l'ensemble des *PD* et di retrouvés,
iv. on retrouve le couple (*z0, dz*) pour lequel les paramètres ci-dessus sont les plus probables.

7. Procédé selon l'une des revendications 1 à 6, **caractérisé en ce que** l'étape 204 comprend des sous-étapes suivantes :
204a : relâchement des contraintes de distance constante entre les *zi* et initialisation avec la solution précédente,
204b : définition des confiances sur les paramètres 3D par la recherche de l'ensemble des *zi, PD, di, f qui* permettent de minimiser l'erreur de re-projection des iris sur les images acquises,
204c : utilisation d'un algorithme du simplexe de type Nelder-Mead / ou un algorithme de recalage de texture additif.

8. Procédé selon l'une quelconque des revendications précédentes, **caractérisé en ce qu'**il comporte une phase 300 de calibrage de la caméra (l'estimation sa distance focale *f*) utilisée pour les acquisitions de données,
ledit calibrage comportant l'utilisation d'un objet prédéterminé et en le présentant devant la caméra, sous diverses distances et orientations angulaires, ledit objet comportant plusieurs cercles concentriques sur un même plan permet de calibrer une caméra ou deux cercles coplanaires.

9. Procédé selon la revendication 8, **caractérisé en ce que** l'objet est un disque compact (CD).

10. Procédé selon l'une des revendications 8 à 9, **caractérisé en ce que** la phase 300 comporte des étapes suivantes :
étape 301 : présentation et déplacement de l'objet devant la caméra,
étape 302 : estimation du centre de l'objet,
étape 303 : détection de contour radial et obtention d'une image de contours avec les contours chainés principaux de l'image,
étape 304 : obtention d'une texture contour/carte de distances au contour de l'objet de face,
étape 305 : utilisation d'un algorithme d'alignement de texture sur l'image des contours pour déterminer les paramètres de calibrage recherchés.

## Patentansprüche

1. Verfahren zur Schätzung der Pupillendistanz (PD) einer Person, das aufweist:
- eine Phase 100 der Datenerfassung, die eine vorbestimmte Bewegung der Person gegenüber einer Kamera verwendet, wobei diese Phase 100 der Datenerfassung die Erfassung einer Einheit von für die Augen der Person repräsentativen aufeinanderfolgenden Bildern aufweist, wenn die Stellung dieser Person sich zwischen einer ersten Konfiguration des Gesichts und einer zweiten Konfiguration des Gesichts ändert,
**dadurch gekennzeichnet, dass** es außerdem aufweist:
- eine Phase 200 der Berechnung von morphologischen und dimensionalen Parametern auf der Basis der erfassten Daten, wobei die Phase 200 folgende Schritte enthält:
Schritt 201: Lernen von statistischen Messungen, wobei der Schritt 201 folgende Teilschritte enthält:
201 a: Messung einer Einheit von morphologischen PD, di, r und mechanischen Daten f, wobei di den Durchmesser der Iris, r den Radius des Augapfels und f die Brennweite der Kamera darstellt
201b: Konstruktion einer Projektionsszene des Systems PD-Iris der Person und Erzeugung einer Entsprechungstabelle, die die kontinuierliche Realität der Parameter gemäß den folgenden Eingaben abtastet: PDpx, dipx, PD, di, f, z, mit z dem Abstand der Mitte der Augen zur Kamera, PDpx dem Abstand zwischen den zwei Mitten der Bilder der Iris und dipx dem Durchmesser der Bilder der Iris, wobei PDpx und dipx den Bildmessungen der Daten PD und di entsprechen,
201 c: Erstellung mehrerer geglätteter diskreter Wahrscheinlichkeitsdichten durch ein Kernverfahren und verteilt in den Tabellen, die unter anderen die folgenden Funktionen ausdrücken:
i. PD = f (PDpx, dipx, f, zi)
ii. di = f (PDpx, dipx, f, zi) wobei zi (i=1...n) die Abstände der Kamera zur Person für die bei der Bewegung erfassten aufeinanderfolgenden Bilder darstellen, n die Anzahl von Bildern darstellt
Schritt 202: Kennzeichnung der Bildmessungen,
Schritt 203: statistische Bestimmung einer metrischen 3D-Schätzung der gesuchten Parameter im wahren Raum des Subjekts: PD, di, f und zi (i=1...n),
Schritt 204 : nicht-lineare Optimierung der Parameter.

2. Verfahren nach Anspruch 1, **dadurch gekennzeichnet, dass** die Phase 100 folgende Schritte enthält:
Schritt 101: Anordnen der Person vor der Kamera in einem vorab gewählten Abstand von der Kamera,
Schritt 102: Bewegung des Gesichts der Person zur Kamera unter Fixierung der Kamera,
Schritt 103: Anhalten, wenn die Person das Objektiv nicht mehr fixieren kann oder wenn sie ein Haltesignal empfängt,
- wobei der vorab gewählte Abstand im Wesentlichen eine Armlänge ist.

3. Verfahren nach einem der Ansprüche 1 bis 2, **dadurch gekennzeichnet, dass** die gesuchten Parameter die Pupillendistanz PD, den Durchmesser der Iris di, die Brennweite der Kamera f, die Abstände zi (i=1...n) der Kamera zur Person für die bei der Bewegung erfassten aufeinanderfolgenden Bilder enthalten.

4. Verfahren nach einem der Ansprüche 1 bis 3, **dadurch gekennzeichnet, dass** der Schritt 202 folgende Teilschritte enthält:
202a: Gesichtserfassung,
202b: Erfassung und Erkennung der Augen,
202c: Feinbeschreibung der Iris durch Schätzung der Parameter des Kreises oder der Ellipse, der/die durch die Projektion der Iris auf dem Bild dargestellt wird,
202d: Ableitung der Werte PDpx, Abstand zwischen den zwei Mitten der Bilder der Iris, und dipx, Durchmessers der Bilder der Iris.

5. Verfahren nach einem der Ansprüche 1 bis 4, **dadurch gekennzeichnet, dass** der Schritt 203 folgende Teilschritte enthält:
203a: unter der Annahme der Bewegung mit konstanter Geschwindigkeit, Ausdruck der Einheit der Parameter zi mit zwei Parametern: z0, der Ausgangsabstand (am weitesten entfernt) und dz, der Abstand zwischen zwei Bildaufnahmen,
203b: Simulation einer Einheit von Durchführungen der Abtastung der Bewegung, um die Parameter f, PD und di bestmöglich und probabilistisch bezüglich der in den Bildern PDpx und dipx beobachteten Messungen zu erklären.

6. Verfahren nach Anspruch 5, **dadurch gekennzeichnet, dass** der Teilschritt 203b folgende Punkte aufweist:
i. für eine Einheit von Ausführungstriplets (z0, dz, f) derart, dass die zi (i=1...n) und f in einem Bereich von für das Protokoll akzeptablen Werten enthalten sind, wird ein Histogramm der Triplets erstellt, für die die meisten Paarantworten (PD, di) aufgrund der Entsprechungsfunktionen vorliegen, und die Durchführungen von (zi, PDpx, dipx) um betrachtete Werte herum variiert werden, um die Durchführungsgesetze der Messungen zu simulieren,
ii. für die maximale Spitze der konstruierten Wahrscheinlichkeiten wird die Brennweite f gefunden,
iii. von f werden PD und di aufgrund der gleichen Tabellen abgeleitet, wie der Mittelwert der Eigenschaften asymptotischer normaler Konvergenz der gewonnenen Erfahrungen der Einheit der gefundenen PD und di,
iv. es wird das Paar (z0, dz) gefunden, für das die obigen Parameter die wahrscheinlichsten sind.

7. Verfahren nach einem der Ansprüche 1 bis 6, **dadurch gekennzeichnet, dass** der Schritt 204 folgende Teilschritte enthält:
204a: Lockern der Zwänge konstanten Abstands zwischen den zi und Initialisierung mit der vorhergehenden Lösung,
204b: Definition der Vertrauen auf die 3D-Parameter durch das Suchen der Einheit der zi, PD, di, f, die es ermöglichen, den Fehler der Rückprojektion der Iris auf die erfassten Bilder zu minimieren,
204c: Verwendung eines Algorithmus des Simplex von der Art Nelder-Mead / oder eines zusätzlichen Algorithmus der Textur-Rückstellung.

8. Verfahren nach einem der vorhergehenden Ansprüche, **dadurch gekennzeichnet, dass** es eine Phase 300 der Kalibrierung der Kamera (die Schätzung ihrer Brennweite f) aufweist, die für die Datenerfassungen verwendet wird,
wobei die Kalibrierung die Verwendung eines vorbestimmten Objekts und seine Präsentation vor der Kamera unter verschiedenen Abständen und Winkelausrichtungen aufweist, wobei das Objekt mehrere konzentrische Kreise in der gleichen Ebene, die das Kalibrieren einer Kamera erlauben, oder zwei koplanare Kreise aufweist.

9. Verfahren nach Anspruch 8, **dadurch gekennzeichnet, dass** das Objekt eine Kompaktdiskette (CD) ist.

10. Verfahren nach einem der Ansprüche 8 bis 9, **dadurch gekennzeichnet, dass** die Phase 300 folgende Schritte aufweist:
Schritt 301: Präsentieren und Verschieben des Objekts vor der Kamera,
Schritt 302: Schätzung der Mitte des Objekts,
Schritt 303: Erfassung der radialen Konturen und Erhalt eines Konturbilds mit den verketteten Hauptkonturen des Bilds,
Schritt 304: Erhalt einer Textur Kontur/Karte von Abständen zur Kontur des gegenüberliegenden Objekts,
Schritt 305: Verwendung eines Algorithmus einer Texturausrichtung auf das Bild der Konturen, um die gesuchten Kalibrierungsparameter zu bestimmen.

## Claims

1. Method for estimating the pupil distance (PD) of an individual, including:
- a phase 100 of acquiring data, using a predetermined movement of the individual facing a video camera, this phase 100 of acquiring data including acquiring a set of successive images representative of the eyes of the individual, when the position of this individual is changing, between a first configuration of the face and a second configuration of the face,
**characterized in that** it also includes:
- a phase 200 of calculating dimensional and morphological parameters on the basis of the acquired data, said phase 200 comprising the following steps:
step 201: learning statistical measurements, said step 201 comprising the following substeps:
201 a: measuring a set of morphological and mechanical data PD, di, r, f, where di is iris diameter, r eyeball radius and f the focal length of the video camera,
201b: constructing a projection scene of the PD-iris system of said individual and creating a lookup table that samples the continuous reality of the parameters via the following inputs: *PDpx, dipx, PD, di, f, z* where *z* is the distance from the centre of the eyes to the video camera, PDpx is the distance between the two centres of the images of the irises and dipx the diameters of the images of the irises, *PDpx* and *dipx* corresponding to the image measurements of the data *PD* and *di*,
201c: establishing a plurality of smoothed discrete probability densities using a kernel method, the probability densities being distributed in tables expressing, among others, the following functions:
i. *PD* = *f(PDpx, dipx, f, zi)*
ii. *di* = *f(PDpx, dipx, f, zi)*
where zi (i=1...n) are the distances from the video camera to the individual for the successive images acquired during the movement, n being the number of images,
step 202: characterizing the image measurements,
step 203: determining statistically a metric 3D estimation of the sought-after parameters in the real space of the subject: PD, di, f and zi (i=1...n),
step 204: optimizing the parameters nonlinearly.

2. Method according to Claim 1, **characterized in that** the phase 100 comprises the following steps:
step 101: positioning the individual facing the video camera, at a predetermined distance from the video camera,
step 102: movement of the face of the individual toward the video camera while fixating thereon,
step 103: stopping when the individual can no longer fixate on the lens or when he receives a signal to stop.
- the distance chosen beforehand being substantially an arm's length.

3. Method according to one of Claims 1 to 2, **characterized in that** the sought-after parameters comprise pupil distance *PD,* iris diameter *di,* the focal length of the camera *f*, the distances *zi* (i=1...n) from the video camera to the individual for the successive images acquired during the movement.

4. Method according to one of Claims 1 to 3, **characterized in that** step 202 comprises the following substeps:
202a: face detection,
202b: detection and recognition of the eyes,
202c: fine description of the irises by estimation of parameters of the circle or of the ellipse represented by the projection of the iris in the image,
202d: deduction of the values *PDpx* (i.e. distance between the two centres of the images of the irises) and *dipx* (i.e. diameters of the images of the irises).

5. Method according to one of Claims 1 to 4, **characterized in that** step 203 comprises the following substeps:
203a: assuming that the movement is at constant speed, expressing all of the parameters *zi* with two parameters: *z0*, the starting distance (i.e. the furthest) and *dz,* the distance between two image captures,
203b: simulating a set of actual samples of the movement in order to explain as best as possible and probabilistically the parameters *f, PD* and *di* with respect to the measurements *PDpx* and *dipx* observed in the images.

6. Method according to Claim 5, **characterized in that** substep 203b includes the following points:
i. for a set of actual triplets (*z0, dz, f*) such that the *zi* (i=1...n) and *f* are comprised in a range of values that are acceptable for the protocol, establishing, by virtue of the lookup functions, a histogram of triplets for which the number of response pairs (*PD, di*) is greatest, and making the actual (*zi, PDpx, dipx*) vary about the values considered in order to simulate the actual laws of the measurements,
ii. finding the focal length *f* for the maximum peak of the constructed probabilities,
iii. from *f*, by virtue of the same tables, deducing *PD* and di to be the average of the asymptomatic normal convergence properties of the experiments carried out of all of the *PD* and di found,
iv. finding the pair (*z0, dz)* for which the above parameters are the most probable.

7. Method according to one of Claims 1 to 6, **characterized in that** step 204 comprises the following substeps:
204a: relaxing the constraints of constant distance between the *zi* and initializing with the preceding solution,
204b: defining the confidences in the 3D parameters by seeking the set of *zi, PD, di, f* that allows the error in the reprojection of the irises to be minimized in the acquired images,
204c: using a simplex or Nelder-Mead algorithm / or an additive texture registration algorithm.

8. Method according to any one of the preceding claims, **characterized in that** it includes a phase 300 of calibrating the video camera (estimating its focal length *f*) used for the data acquisition,
said calibration including the use of a preset object and the presentation thereof in front of the video camera, at various distances and angular orientations, said object including a plurality of concentric circles in a given plane allows a video camera to be calibrated or two coplanar circles.

9. Method according to Claim 8, **characterized in that** the object is a compact disc (CD).

10. Method according to one of Claims 8 to 9, **characterized in that** the phase 300 includes the following steps:
step 301: presentation and movement of the object in front of the video camera,
step 302: estimation of the centre of the object,
step 303: radial edge detection and obtainment of an image of edges with the main chained edges of the image,
step 304: obtainment of an edge texture/map of distances of the edge of the object front-on,
step 305: use of a texture alignment algorithm on the image of the edges to determine the sought-after calibration parameters.
